# EUROPÄISCHE PATENTANMELDUNG

(11) **EP 4 035 697 A1**
(43) Veröffentlichungstag der Anmeldung: **03.08.2022**
(21) Anmeldenummer: 22153351.6
(22) Anmeldetag: 26.01.2022
(51) Int. Cl.: A61L 9/20, F24F 1/00

(54) **VERFAHREN UND VORRICHTUNG ZUM DESINFIZIEREN UND STERILISIEREN VON LUFT**

(30) Priorität: 27.01.2021 DE 102021101793
(71) Anmelder: ReTecCom GmbH, 09221 Neukirchen/Erzgebirge (DE)
(72) Erfinder: Hoyer, Daniel, 09127 Chemnitz (DE); Hainzinger, Manfred, 85077 Manching (DE)
(74) Vertreter: Canzler & Bergmeier Patentanwälte Partnerschaft mbB

(57) **Zusammenfassung**

Ein Verfahren zum Desinfizieren und Sterilisieren von Luft hindert Strahlung von UV-Lampen (9) am Austreten aus einer Kammer (K) durch eine mechanische Abschirmung und durch absorbierende Oberflächen (O) im Bereich des Einlasses (E) und/oder des Auslasses (A). Eine entsprechende Vorrichtung zum Desinfizieren und Sterilisieren von Luft, weist eine Kammer (K) mit Seitenwänden (1, 1'), einer Bodenwand (2) und einer Deckwand (13) auf, wobei die Kammer (K) einen Innenraum (I), einen Einlass (E) und einen Auslass (A) umfasst, der Kammer (K) ein Gebläse zugeordnet ist, in dem Innenraum (I) der Kammer (K) aus zumindest einer Leitwand (8) ein Labyrinth gebildet ist, das einen mäandrierenden Strömungsweg (S) für die Luft bildet, in dem Strömungsweg (S) mehrere UV-Lampen (9) angebracht sind, wobei die UV-Lampen (9) in Richtung des Strömungsweges (S) ausgerichtet sind, sodass die Luft entlang der UV-Lampen (9) strömt. Der Kammer (K) ist ein Sichtschutz zugeordnet, um eine Sicht auf die UV-Lampen (9) durch den Einlass (E) und den Auslass (A) zu verhindern und eine Innenseite der Kammer (K) weist im Bereich des Einlasses (E) und/oder des Auslasses (A) eine absorbierende Oberfläche (O) auf, um eine Reflexion der Strahlung der UV-Lampen (9) aus der Kammer (K) zu verhindern.

## Beschreibung

Die vorliegende Erfindung betrifft ein Verfahren zum Desinfizieren und Sterilisieren von Luft, wobei die Luft durch einen Einlass in einen Innenraum einer Kammer einer Vorrichtung eingesaugt wird, die Luft in dem Innenraum in einem mäandrierenden Strömungsweg entlang von mehreren UV-Lampen strömt und von den UV-Lampen bestrahlt wird und anschließend den Innenraum der Kammer durch einen Auslass wieder verlässt, sowie eine Vorrichtung zum Desinfizieren und Sterilisieren von Luft, bei der eine Kammer mit Seitenwänden, einer Bodenwand und einer Deckwand ausgebildet ist, wobei die Kammer einen Innenraum, einen Einlass und einen Auslass, umfasst, der Kammer ein Gebläse zugeordnet ist, in dem Innenraum der Kammer aus zumindest einer Leitwand ein Labyrinth gebildet ist, das einen mäandrierenden Strömungsweg für die Luft bildet, in dem Strömungsweg mehrere UV-Lampen angebracht sind und wobei die UV-Lampen in Richtung des Strömungsweges ausgerichtet sind, so dass die Luft entlang der UV-Lampen strömt.

Aus der DE 100 84 820 T 1 ist ein Verfahren und eine Vorrichtung zur Reinigung eines sauerstoffhaltigen Gases bekannt. Die beschriebene Vorrichtung zur Reinigung von Luft umfasst einen ersten Behandlungsraum mit Mitteln für die Zufuhr der Luft und einer Einrichtung zur Erzeugung von Ozon in der Luft, z.°B. mit einer Einrichtung zur Bestrahlung der Luft mit UV-Strahlen kurzer Wellenlänge von 110 nm oder länger, aber kürzer als 200 nm, einen zweiten Behandlungsraum, der mit dem ersten Behandlungsraum verbunden ist und eine Einrichtung zur Bestrahlung der aus dem ersten Behandlungsraum zugeführten Luft mit UV-Strahlen mittlerer Wellenlänge von 200 nm oder länger, aber kürzer als 300 nm aufweist, einen dritten Behandlungsraum, der mit dem zweiten Behandlungsraum verbunden ist und eine Einrichtung zum Bestrahlen der aus dem zweiten Behandlungsraum zugeführten Luft mit UV-Strahlen langer Wellenlänge von 300 nm oder länger, aber kürzer als 380 nm aufweist, und Mittel zum Ablassen der im dritten Behandlungsraum behandelten Luft aus der Vorrichtung, wobei der zweite und/oder der dritte Behandlungsraum einen Photokatalysator aufweist. Der Photokatalysator haftet an den Trennwänden und den Innenwänden des Gehäuses im zweiten und dritten Behandlungsraum an oder ist darauf aufgetragen. Als Photokatalysator wurden Teilchen aus Titanoxid mit orthorhombischem Kristallsystem (Teilchen aus Brookit) angebracht. Weiterhin ist eine IR-Lampe und eine Halogenlampe in einem Trockenraum installiert. Mit dieser Vorrichtung soll eine große zu behandelnde Luftmenge unverzüglich desinfiziert, desodoriert und gereinigt werden. Nachteilig bei dieser Vorrichtung ist der komplizierte Aufbau sowie die Gefahr, dass Strahlung in der Vorrichtung nach außen dringen kann und somit Personen in der Nähe dieser Vorrichtung geschädigt werden können. Außerdem ist zwischen den einzelnen Behandlungsräumen jeweils ein Kanal vorgesehen, in welchem die Luft unbehandelt bleibt, wohl um die unterschiedlichen Behandlungsräume voneinander zu trennen. Dies führt zu einer Schwächung der Dekontaminierung.

Aufgabe der vorliegenden Erfindung ist es somit ein Verfahren und eine Vorrichtung zum Desinfizieren und Sterilisieren von Luft zu schaffen, welche einen hohen Wirkungsgrad, insbesondere in Bezug auf die Abtötung von Viren, vorzugsweise Coronaviren, hat und dabei sicherstellt, dass die hierfür erforderliche harte Strahlung Personen, welche sich in der Nähe der Vorrichtung befinden, nicht schädigt.

Vorgeschlagen wird ein Verfahren zum Desinfizieren und Sterilisieren von Luft, wobei die Luft durch einen Einlass in einen Innenraum einer Kammer einer Vorrichtung eingesaugt wird. Die Luft strömt in dem Innenraum in einem mäandrierenden Strömungsweg entlang von mehreren UV-Lampen. Durch den mäandrierenden Strömungsweg, in welchem sich die UV-Lampen befinden und die entlangströmende Luft von den UV-Lampen bestrahlt wird, wird sichergestellt, dass die zu behandelnde Luft möglichst ständig und lange den UV-Lampen ausgesetzt ist. Je länger die Luft Kontakt mit der Strahlung der UV-Lampen hat, desto höher wird der Reinigungsgrad sein.

Insbesondere bei der Abtötung von Viren, vorzugsweise Coronaviren, ist eine sehr intensive Bestrahlung der mit Viren kontaminierten Luft erforderlich. Es ist dabei wichtig, dass die kontaminierte Luft ausreichend lange der Strahlung ausgesetzt ist. Dadurch, dass die Luft in jedem Abschnitt des mäandrierenden Strömungswegs der Strahlung ausgesetzt ist, wird eine sehr intensive und effektive Abtötung der Viren und auch beispielsweise von Bakterien, bewirkt. Dadurch, dass die UV-Lampen im Wesentlichen komplett ihrer Länge nach umströmt werden, ist eine besonders gute Wirkung festzustellen.

Die Luft verlässt anschließend den Innenraum der Kammer durch einen Auslass. Wichtig ist es hierbei, dass zwar einerseits die Luft möglichst ungehindert aus der Umgebung in die Vorrichtung hinein und aus der Vorrichtung wieder hinausströmen kann, die Strahlung allerdings im Inneren der Vorrichtung bleibt. Insbesondere harte Strahlung, welche für die Abtötung von Coronaviren, wie beispielsweise COVID-19-Viren, erforderlich ist, kann Personen, welche sich in der Nähe der Vorrichtung befinden, nachhaltig schädigen. Soll die Vorrichtung zur Reinigung von Wohn- oder Arbeitsräumen eingesetzt werden, so sind Einrichtungen, welche Strahlung aus der Vorrichtung austreten lassen, ungeeignet. Erfindungsgemäß wird bei dem Verfahren zum Desinfizieren und Sterilisieren von Luft die Strahlung der UV-Lampen am Austreten aus der Kammer durch zumindest eine mechanische Abschirmung gehindert. Die mechanische Abschirmung sorgt dabei dafür, dass die Strahlung möglichst nicht direkt aus den Öffnungen am Einlass und am Auslass austreten kann. Sie sorgt somit für eine Art Sichtschutz, damit die UV-Lampen von außen nicht sichtbar sind. Um zusätzlich zu vermeiden, dass durch Reflexionen die Strahlung dennoch durch den Einlass und/oder den Auslass austreten kann, ist durch eine absorbierende Oberflächenbeschichtung im Bereich des Einlasses und/oder des Auslasses sichergestellt, dass die Strahlung in diesen Bereichen absorbiert wird. Reflexionen der Strahlung werden somit auch davon abgehalten, dass sie aus der Vorrichtung austreten könnten. Mit dem erfindungsgemäßen Verfahren wird somit sichergestellt, dass einerseits die Viren, mit welcher Luft in einem Wohn- oder Arbeitsraum kontaminiert ist, sehr umfassend vernichtet werden und andererseits Personen, welche sich in der Nähe der mit dem Verfahren arbeitenden Vorrichtung befinden, bestmöglich vor der Strahlung in dem Gerät geschützt werden. Außerdem wird durch den mäandrierenden Strömungsweg, in welchem sich die UV-Lampen befinden, eine kompakte Bauweise und dennoch ein langer Bestrahlungsweg und damit eine lange Bestrahlungsdauer der Luft ermöglicht.

Von Vorteil ist es, wenn die Luft in dem Innenraum mehr als 1 Sekunde, vorzugsweise mehr als 1,75 Sekunden verweilt. Je länger die Luft in dem Innenraum der Strahlung der UV-Lampen ausgesetzt sein kann, desto effektiver ist die Dekontaminierung der Luft von den Viren. Nachdem die Luft die Vorrichtung, insbesondere den Innenraum der Kammer durchströmt, kann entweder der Strömungsweg verlängert werden oder die Geschwindigkeit, mit welcher die Luft durch die Vorrichtung strömt, reduziert werden, um eine möglichst lange Bestrahlungszeit zu ermöglichen. Eine Bestrahlung von mehr als einer Sekunde, vorzugsweise mehr als 1,75 Sekunden, hat sich dabei als Zeitdauer herausgestellt, welche in den meisten Fällen eine ausreichende Abtötung der in der Luft enthaltenen Viren und Bakterien ermöglicht. In Verbindung mit der mäandrierenden Durchströmung der Vorrichtung, kann eine kompakte Bauform erreicht werden und dennoch muss nicht auf eine ausreichend lange Bestrahlung verzichtet werden. Dies ist besonders vorteilhaft für den Einsatz der Vorrichtung in Wohnräumen oder Arbeitsräumen, wie beispielsweise Büros oder Schulräumen.

Vorteilhafterweise strahlen die UV-Lampen als UVC-Lampen mit einer Wellenlänge zwischen 100 bis 280 nm, vorzugsweise zwischen 220 bis 280 nm. Viren werden in diesem Bereich besonders nachhaltig geschädigt, weshalb sich UVC-Lampen besonders gut zur Zerstörung von Viren in der Luft eignen. Allerdings sind die von UVC-Lampen ausgesandten Wellenlängen der Strahlung sehr schädlich für Menschen, insbesondere für die Hornhaut der Augen. Die erfindungsgemäße Abschirmung der Vorrichtung ist daher besonders wichtig.

Auch ist es von Vorteil, wenn in der Vorrichtung eine Bestrahlungsdosis H von mindestens 50 J/m², vorzugsweise von mehr als 110 J/m² erzeugt wird. Je höher die Bestrahlungsdosis ist, desto effektiver werden die Viren zerstört. Dementsprechend ist auch die erforderliche Bestrahlungszeit für eine nahezu vollständige Abtötung der Viren entsprechend kürzer, wenn eine höhere Bestrahlungsdosis gewählt wird. Die Luft kann dann bei einer vorgegebenen erfindungsgemäßen Vorrichtung entsprechend schneller durch die Vorrichtung hindurchbewegt werden, wodurch auch ein vorbestimmtes Luftvolumen in den Raum, in welchem die Vorrichtung aufgebaut ist, schneller ausgetauscht werden kann.

Ebenso ist es vorteilhaft, wenn die Luft in dem Innenraum verwirbelt wird. Durch die Verwirbelungen der Luft in dem Innenraum der Vorrichtung wird gewährleistet, dass alle Luftpartikel möglichst lange in Kontakt mit der Strahlung der UV-Lampen kommen. Damit ist die nahezu vollständige Abtötung der in der Luft enthaltenen Viren zu erzielen.

Eine erfindungsgemäße Vorrichtung zum Desinfizieren und Sterilisieren von Luft, weist eine Kammer mit Seitenwänden, einer Bodenwand und einer Deckwand auf, wobei die Kammer einen Innenraum, einen Einlass und einen Auslass umfasst. Der Kammer ist ein Gebläse zugeordnet, mit welchem die Luft aus der Umgebung der Vorrichtung angesaugt, durch die Vorrichtung hindurch und aus der Vorrichtung wieder herausgeblasen werden kann. In dem Innenraum der Kammer ist aus zumindest einer Leitwand ein Labyrinth gebildet, das einen mäandrierenden Strömungsweg für die Luft bildet. Trotz kompakter Bauweise der Vorrichtung ist es damit möglich, dass die Luft einen langen Weg zwischen Einlass und Auslass zurücklegen muss. Der Mäander bildet eine Folge von starken, die Richtung wechselnden Windungen. Durch den kurvenreichen, vielgewundenen Verlauf des Strömungsweges wird die hindurchströmende Luft verwirbelt und gemischt und benötigt durch die entsprechende Länge des Strömungsweges eine relativ lange Zeit, die mehr als zehnmal so lange dauern kann wie der direkte Durchgang der Luft vom Einlass bis zum Auslass der Vorrichtung.

In dem Strömungsweg sind mehrere UV-Lampen angebracht, wobei die UV-Lampen in Richtung des Strömungsweges, von einer ersten Umlenkung des Strömungsweges bis zur nächsten Umlenkung des Strömungsweges ausgerichtet sind, so dass die Luft entlang der UV-Lampen strömt. Damit wird bewirkt, dass die Luft möglichst lange, vorzugsweise während der gesamten Durchströmung durch den Innenraum der Vorrichtung der unmittelbaren Strahlung der UV-Lampen ausgesetzt ist.

Um zu gewährleisten, dass die Strahlung der UV-Lampen nicht nach außen aus der Vorrichtung dringt, ist der Kammer ein Sichtschutz zugeordnet. Hierdurch wird eine Sicht auf die UV-Lampen durch den Einlass und den Auslass verhindert. Der Sichtschutz umfasst vorzugsweise zumindest eine mechanische Abschirmung, beispielsweise weitere Trennwände, welche zwischen den Öffnungen des Einlasses und/Auslasses für die Luft angeordnet ist.

Zusätzlich zu diesem Sichtschutz weist eine Innenseite der Kammer im Bereich des Einlasses und/oder des Auslasses eine absorbierende Oberfläche auf, um eine Reflexion der Strahlung der UV-Lampen aus der Kammer zu verhindern. Die absorbierende Oberfläche kann durch eine entsprechende Strukturierung der Oberfläche der Wände im Bereich des Einlasses und/oder des Auslasses erfolgen. Ebenso ist es möglich, dass eine absorbierende Wirkung der Oberfläche dadurch entsteht, dass die Wände im Bereich des Einlasses und/oder des Auslasses mit einer Oberflächenbeschichtung, beispielsweise einer matten Farbe, vorzugsweise einer strukturierenden Farbe, insbesondere schwarz, beschichtet sind. Die Vorrichtung ist somit in der Lage, unabhängig von ihrer Installationsart und -höhe und der Geräteanzahl im Raum, die tatsächliche Strahlenbelastung für Personen unkritisch gering zu halten.

Auch ist es von Vorteil, wenn die Innenseite der Kammer im Bereich des Innenraums eine reflektierende Oberfläche aufweist. Im Gegensatz zu der absorbierenden Oberfläche im Bereich des Einlasses und/oder des Auslasses ist im Bereich des Innenraums eine reflektierende Oberfläche vorteilhafter. Durch die reflektierende Oberfläche wird die Strahlung der UV-Lampen reflektiert und verstärkt somit die direkte Bestrahlung der Luft durch eine weitere indirekte Bestrahlung. Inseln, in welcher die Luft während ihres Weges durch die Vorrichtung unbestrahlt bleiben würde, entstehen somit nicht. Die Luft wird in dem Strömungsweg somit allseitig und stets bestrahlt, auch wenn sie sich, beispielsweise im Bereich einer Windung des Strömungsweges, also nicht unmittelbar neben einer UV-Lampe befindet.

Vorteile bringt es mit sich, wenn die UV-Lampen zwischen benachbarten Leitwänden angeordnet sind. Die Leitwände bilden den Strömungsweg ab, entlang dessen sich die Luft zwischen dem Einlass und dem Auslass bewegt. Durch die Anordnung der UV-Lampen zwischen benachbarten Leitwänden ist gewährleistet, dass die UV-Lampen weitgehend vollständig und allseitig von der Luft umströmt werden. Die Bestrahlung der Luft erfolgt somit lückenlos und langandauernd. Damit wird auch gewährleistet, dass es keine Luftvolumina gibt, welche während der Durchströmung der Vorrichtung nur kurzzeitig bestrahlt worden wären, und es somit Viren ermöglichen würde zu überleben.

Vorteilhaft ist es, wenn sich die Leitwand zwischen der Bodenwand und der Deckwand erstreckt. Damit wird sichergestellt, dass die Luft tatsächlich den kompletten Strömungsweg durchströmen muss und nicht über eine Leitwand hinweg den Weg abkürzen kann. Die komplette Luft wird somit gleichmäßig und für die gleiche Zeitdauer bestrahlt.

Ebenso ist es vorteilhaft, wenn die Leitwand zwei Enden aufweist, wobei eines der Enden an eine der Seitenwände anschließt und das andere Ende frei in den Strömungsweg ragt. Die Leitwand bildet somit mit ihren beiden Seitenflächen eine Begrenzung des Strömungsweges zwischen zwei parallel zueinander angeordneten UV-Lampen. Der Strömungsweg und die UV-Lampen sind damit im Wesentlichen quer zu der Vorrichtung bzw. der Kammer angeordnet. Die Umkehrstellen an den Windungen sind nahe der Seitenwände angeordnet. Die Konstruktion der Vorrichtung wird hierdurch einfach und die Vorrichtung kann kompakt ausgebildet werden.

Vorteilhaft ist es, wenn zumindest eines der Enden der Leitwand abgewinkelt ist. Die Abwinkelung der Leitwand verhindert einerseits, dass Bereiche in den Strömungsweg entstehen, welche eine stehende Luft aufweisen, welche nicht durch die Vorrichtung hindurchgefördert wird. Andererseits wird durch die Abwinkelung der Leitwand auch eine Verwirbelung der Luft erzeugt, wodurch ebenfalls gewährleistet wird, dass die komplette Luft gleichmäßig von den UV-Lampen bestrahlt werden kann.

Vorteile bringt es mit sich, wenn das freie Ende der Leitwand eine Verwirbelungskante aufweist. Die Verwirbelungskante, welche beispielsweise auch als Abwinkelung der Leitwand ausgebildet sein kann, bewirkt, dass die Luft verwirbelt und gemischt wird, sodass wiederum eine gleichmäßige Bestrahlung durch die UV-Lampen bewirkt wird.

Ebenso ist es vorteilhaft, wenn an einer der Seitenwände das Gebläse, vorzugsweise mehrere Ventilatoren umfassend, angeordnet ist. Das Gebläse, das vorzugsweise am Einlass der Vorrichtung angeordnet ist, fördert Luft in die Vorrichtung hinein. Durch den erzeugten Luftdruck wird die Luft durch die Vorrichtung hindurch befördert und tritt am Auslass an einer entsprechenden Auslassöffnung aus der Vorrichtung wieder aus. Die Vorrichtung verfügt somit über eine aktive Luftumwälzung, wobei sich an den Stirnseiten flächige Lufteintritts- und Austrittsflächen befinden.

Vorteilhaft ist es, wenn am Einlass und/oder am Auslass Lamellen angeordnet sind, um die Luft gleichmäßig in und/oder aus dem Innenraum zu fördern. Durch die Lamellen am Einlass wird bewirkt, dass eine ausreichende Luftmenge einströmen kann, ohne sich aufzustauen oder gar wieder aus der Vorrichtung heraus zu strömen, ohne den Strömungsweg durchströmt zu haben. Die Lamellen am Auslass bewirken, dass die Luft gleichmäßig und vorzugsweise ohne einen starken Luftzug zu generieren, aus der Vorrichtung ausströmen kann. Dies erhöht den Komfort der Vorrichtung für Personen, welche sich in der Nähe der Vorrichtung befinden.

In einer besonders bevorzugten Ausführung der Lamellen sind diese in ihrer Neigung verstellbar. Hierdurch ist es möglich, dass die Richtung, aus welcher die Luft angesaugt wird bzw. in welche die Luft ausgeblasen wird, einstellbar ist. Auf die individuellen Anforderungen an den Aufstellungsort der Vorrichtung kann hierdurch optimal eingegangen werden. Durch die Lamellen am Auslass, insbesondere, wenn diese in ihrer Neigung verstellbar sind, ist zudem zu beeinflussen, wie stark der Rückstau des Luftstroms in der Vorrichtung ist. Hierdurch kann auch die Aufenthaltsdauer der Luft in der Vorrichtung beeinflusst werden. Indem die Lamellen beispielsweise schräg zum Luftstrom gestellt werden und somit einen höheren Widerstand für den Luftstrom bilden, wird die Luft eher zurückgestaut und benötigt länger, um die Vorrichtung wieder zu verlassen. Sind die Lamellen hingegen in Richtung des Luftstroms gestellt, so bilden sie weniger Widerstand für die Luft, sodass diese die Vorrichtung schneller verlassen kann.

Vorteilhaft ist es zudem, wenn die Lamellen eine absorbierende Oberfläche aufweisen. Durch die absorbierende Oberfläche der Lamellen, welche wiederum durch eine Beschichtung und/oder eine Struktur erfolgen kann, wird zusätzlich und in Kombination zu den anderen Maßnahmen verhindert, dass die Strahlung der UV-Lampen aus der Vorrichtung austreten kann und Personen schädigen könnte.

Auch ist es vorteilhaft, wenn die UV-Lampen UVC-Lampen mit einer Wellenlänge zwischen 100 bis 280 nm, vorzugsweise zwischen 220 bis 280 nm sind. Diese UVC-Lampen sind besonders gut dafür geeignet Viren, insbesondere Coronaviren, abzutöten. Weisen die UVC-Lampen eine Wellenlänge zwischen 220 bis 280 nm, ganz bevorzugt etwa 250 nm, auf, so ist die Effektivität der Vorrichtung in Bezug auf die Abtötung der Coronaviren am Optimalsten.

Auch ist es von Vorteil, wenn die UV-Lampen, insbesondere die UVC-Lampen, in der Vorrichtung eine Bestrahlungsdosis H von mindestens 50 J/m², vorzugsweise von mehr als 110 J/m² erzeugen. Je höher die Bestrahlungsdosis ist, desto stärker ist der Effekt der Abtötung der Coronaviren.

Durch das Zusammenwirken der Strömungsgeschwindigkeit der Luft, welche durch das Gebläse erzeugt wird, der Wellenlänge der UV-Lampen, insbesondere der UVC-Lampen, und der Bestrahlungsdosis ist das jeweilige Luftvolumen so lange mit der Strahlung der UV-Lampen in Kontakt, dass nahezu alle darin enthaltenen Viren, insbesondere Coronaviren, zerstört werden. Beste Ergebnisse wurden erzielt mit einer Verweilzeit der Luft im Einflussbereich der UV-Lampen von etwa 2 Sekunden, einer Wellenlänge der UVC-Lampen von etwa 250 nm und einer Bestrahlungsdosis von mehr als 110 J/m².

Das Verfahren und die Vorrichtung sind gemäß der vorangegangenen Beschreibung ausgebildet, wobei die genannten Merkmale einzeln oder in beliebiger Kombination vorhanden sein können.

Weitere Vorteile der Erfindung sind in den nachfolgenden Ausführungsbeispielen beschrieben. Es zeigt:
- **Figur 1**: eine Draufsicht auf eine geöffnete erfindungsgemäße Vorrichtung und
- **Figur 2**: eine Seitenansicht auf die Vorrichtung gemäß Figur 1, ohne Seitenwand.

Bei der nachfolgenden Beschreibung der in den Figuren dargestellten alternativen Ausführungsbeispiele werden für Merkmale, die im Vergleich zu den zuvor beschriebenen Ausführungsbeispielen in ihrer Ausgestaltung und/oder Wirkweise identisch und/oder zumindest vergleichbar sind, gleiche Bezugszeichen verwendet. Sofern diese nicht nochmals detailliert erläutert werden, entspricht deren Ausgestaltung und/oder Wirkweise der Ausgestaltung und Wirkweise der vorstehend bereits beschriebenen Merkmale. Aus Übersichtlichkeitsgründen kann es sein, dass nicht alle gleichen Teile mit Bezugszeichen versehen, aber gleichartig gezeichnet sind.

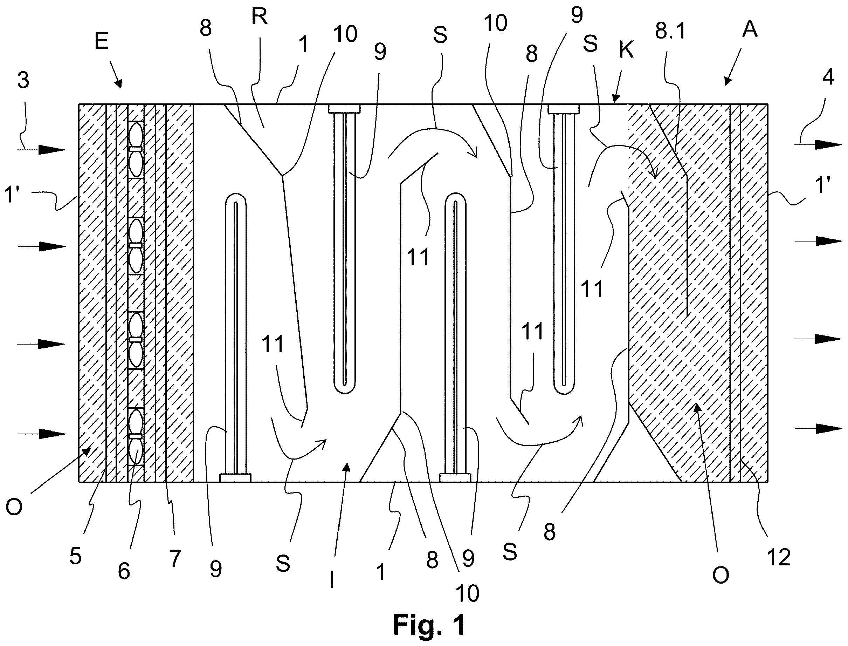

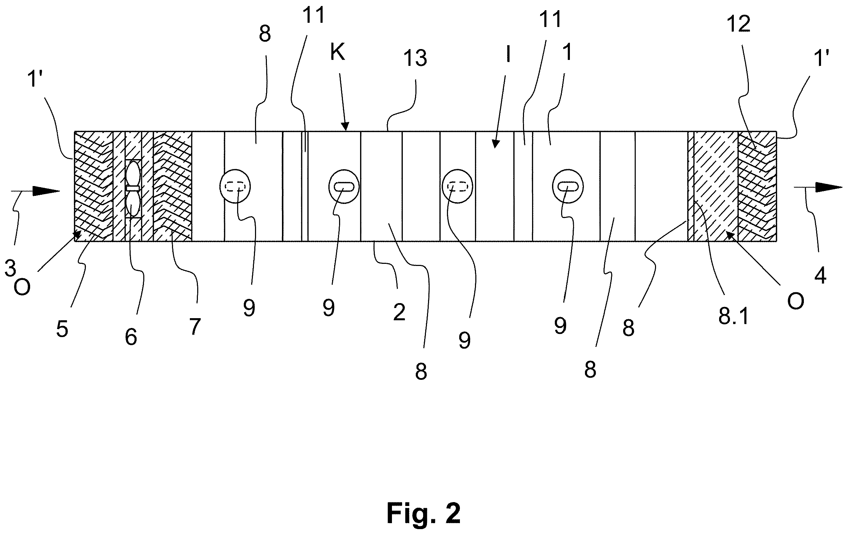

Figur 1 zeigt eine Draufsicht auf eine geöffnete erfindungsgemäße Vorrichtung. Die Vorrichtung weist eine Kammer K mit vier Seitenwänden 1 und 1', einer Bodenwand 2 sowie einer hier nicht dargestellten Deckwand 13 (siehe Figur 2) auf. Zwei der Seitenwände 1 sind entlang der Längsseite der Kammer K angeordnet, während sich die beiden anderen Seitenwände 1' an den Stirnseiten der Kammer K befinden. An der links dargestellten Stirnseite der Kammer K zeigen Pfeile 3 an, dass Luft in die Kammer K eintreten kann. Diese Seitenwand 1' stellt somit einen Einlass E dar. An der gegenüberliegenden, rechts dargestellten Stirnseite der Kammer K deuten die Pfeile 4 an, dass an dieser Seitenwand 1' die in der Kammer K behandelte Luft aus der Kammer K wieder austritt. Diese stirnseitige Seitenwand 1' der Kammer K entspricht somit einem Auslass A.

Im Bereich des Einlasses E sind erste Lamellen 5 vorgesehen. Diese Lamellen 5 sind gemäß einer vorteilhaften Ausführung der Erfindung in ihrer Neigung verstellbar, so dass die Ansaugrichtung der Luft einstellbar ist. Dies ist vorteilhaft wenn die Vorrichtung beispielsweise an einer Zimmerdecke installiert ist, um Luft von unterhalb der Vorrichtung besser in die Vorrichtung einsaugen zu können. Bei einer anderen Aufstellung der Vorrichtung kann es hingegen vorteilhafter sein, wenn die Luft aus einer anderen Richtung angesaugt wird, wobei in diesem Fall die Lamellen 5 in ihrer Neigung entsprechend verstellt werden können.

Im Anschluss an die Lamellen 5 ist in den Bereich des Einlasses E eine Leiste mit mehreren Ventilatoren 6 angeordnet. Die Luft wird durch die Ventilatoren 6 in Richtung der Pfeile 3 in die Vorrichtung eingesaugt. Die Geschwindigkeit der Ventilatoren und die Stellung der Lamellen 5 bestimmen die Geschwindigkeit, mit welcher die Luft in die Vorrichtung eingesaugt wird. Um bei Bedarf trotz einer bestimmten Geschwindigkeit der Ventilatoren 6 eine Reduzierung der Luftgeschwindigkeit vornehmen zu können, sind im Anschluss an die Ventilatoren 6, weiterhin in dem Bereich des Einlasses E, zweite Lamellen 7 vorgesehen. Die zweiten Lamellen 7 sind vorzugsweise ebenfalls in ihrer Neigung verstellbar. Hierdurch kann der Luftwiderstand beeinflusst werden, wodurch die Geschwindigkeit und Menge der eingesaugten Luft bei Bedarf reduziert wird. Durch die reduzierte Luftgeschwindigkeit kann die Aufenthaltsdauer der Luft in der Vorrichtung verlängert werden. Hierdurch ist die Bestrahlung der Luft über einen längeren Zeitraum gegeben, wodurch die Reinigung und die Dekontaminierung der Luft verbessert wird.

Nach dem Durchströmen des Einlasses E tritt die Luft in einen Innenraum I der Kammer K ein. In dem Innenraum I erfolgt die Abtötung der Viren und dadurch die Dekontaminierung und somit die Desinfizierung und Sterilisierung der Luft. Die einströmende Luft wird in dem Innenraum I in einem mäanderförmigen Strömungsweg mehrfach umgelenkt. Durch diese Umlenkung verlängert sich der Strömungsweg im Vergleich zu einer geradlinigen Durchströmung der Vorrichtung bzw. des Innenraums I um ein Mehrfaches. Hierdurch wird bewirkt, dass die Behandlungsdauer der Luft ebenfalls entsprechend verlängert werden kann. Je länger die Luft in dem Innenraum I behandelt werden kann, desto umfänglicher ist die Vernichtung von in der Luft enthaltenen Viren und anderen Schadstoffen.

Die mäanderförmige Umlenkung der Luft erfolgt mit Leitwänden 8. Die Leitwände 8 sind abwechselnd mit einem ihrer Enden an der einen und an der anderen Seitenwand 1 befestigt. Sie reichen von der Bodenwand 2 bis zur Deckwand 13 (siehe Figur 2) und leiten somit die einströmende Luft vollständig entlang des durch die Leitwände 8 definierten Strömungsweges. Dies ist durch die Pfeile S angedeutet. Die Luft strömt dementsprechend in Art eines Labyrinths quer zur Kammer K, wird im Bereich einer der beiden Seitenwände 1 umgelenkt und strömt wieder quer zur Kammer K bis zur gegenüberliegenden Seitenwand 1. Bei der vorliegend dargestellten Kammer K wird die Luft viermal umgelenkt. Die Länge des Strömungsweges entspricht somit etwa der vierfachen Breite der Kammer K bzw. Länge der stirnseitig angeordneten Seitenwände 1'. In dem Strömungsweg und zwischen den Lamellen 7 und einer Leitwand 8 bzw. den benachbarten Leitwänden 8 sind UV-Lampen 9 angeordnet. Die UV-Lampen 9 sind in Richtung des Strömungsweges, quer zur Längsrichtung der Kammer K ausgerichtet, sodass die Luft entlang der UV-Lampen 9 strömt. Diese UV-Lampen 9 sind vorzugsweise UVC-Lampen mit einer Wellenlänge zwischen 100 bis 280 nm, vorzugsweise zwischen 220 bis 280 nm. Es hat sich nämlich herausgestellt, dass Viren, insbesondere Coronaviren, mit einer Wellenlänge von etwa 250 nm am effektivsten abzutöten sind. Dadurch, dass die kontaminierte Luft mit den darin enthaltenen Viren entlang des kompletten Strömungsweges einer entsprechenden Strahlung mit dieser Wellenlänge vor allem direkt, aber auch indirekt durch Reflektion, ausgesetzt ist, ist es möglich, dass trotz des relativ einfachen und kompakten Aufbaus der Kammer K bzw. der gesamten Vorrichtung eine Abtötung von über 99 Prozent der in der Luft enthaltenen Coronaviren möglich ist.

Die UV-Lampen 9, insbesondere die UVC-Lampen, erzeugen eine Bestrahlungsdosis H von mindestens 50 J/m², vorzugsweise von mehr als 110 J/m². Hierdurch wird die Luft sehr intensiv bestrahlt und die Kontamination der Luft mit Viren und Bakterien sehr effektiv beseitigt. Je höher die Bestrahlungsdosis ist, desto kürzer ist die erforderliche Aufenthaltsdauer der Luft in dem Innenraum I der Kammer K, um möglichst alle Viren und Bakterien zu zerstören.

Um die in dem Innenraum I strömende Luft vollständig den UV-Lampen 9 aussetzen zu können, sind die Leitwände 8 mit Abwinkelungen 10 versehen. Durch die Abwinkelungen 10 wird sichergestellt, dass keine Luftvolumina in Todräumen verharren, dadurch unter Umständen nicht ausreichend bestrahlt werden und insbesondere die Durchsatzmenge der Luft durch die Kammer K verringern.

An den freien Enden der Leitwände 8 sind Verwirbelungskanten 11 vorgesehen. Die Verwirbelungskanten 11 sorgen dafür, dass die hindurchströmende Luft immer wieder verwirbelt wird und dadurch gewährleistet wird, dass die komplette Luft gleichmäßig durch die UV-Lampen 9, entlang denen die Luft strömt, bestrahlt wird. Die Strömung reist an den Verwirbelungskanten 11 ab und sorgt dadurch für eine Durchmischung der Luft.

Um die Wirkung der UV-Lampen 9 gegenüber der entlang strömenden Luft besonders effektiv gestalten zu können, sind die Oberflächen der Bodenwand 2, der Deckwand 13, der Seitenwände 1 sowie der Leitwände 8 in dem Innenraum I mit einer reflektierenden Oberfläche R ausgestattet. Durch die Reflexionen der UV-Strahlung wird die durchströmende Luft allseitig bestrahlt, wodurch die darin enthaltenen Viren und anderen Verunreinigungen, wie beispielsweise Bakterien, weitgehend vollständig vernichtet werden können.

Nach dem Passieren der letzten UV-Lampen 9 gelangt die Luft in eine letzte Passage im Bereich des Auslasses A. Hier ist eine letzte Leitwand 8.1 angeordnet, welche gleichzeitig, hier zusammen mit der letzten Leitwand 8 des Innenraums I einen Sichtschutz bildet, sodass von der ausgangsseitigen Seitenwand 1' kein Blick auf die letzte UV-Lampe 9 des Innenraums I ermöglicht wird. Nach dieser letzten Passage zwischen den Leitwänden 8 und 8.1 gelangt die Luft durch Lamellen 12 aus der Kammer K hinaus. Wenn die Luft die Kammer K verlässt sind nahezu alle Viren und Bakterien, welche sich in der Luft befunden haben, abgetötet und die Luft gilt dementsprechend als desinfiziert und sterilisiert.

Der Kammer K ist ein Sichtschutz zugeordnet, um eine Sicht auf die UV-Lampen 9 durch den Einlass E und den Auslass A zu verhindern. Dieser Sichtschutz ist im Bereich des Einlasses E durch die Lamellen 5 und 7 sowie die Leiste mit den Ventilatoren 6 gebildet. Im Bereich des Auslasses A bilden die letzte Leitwand 8 des Innenraums I zusammen mit der Leitwand 8.1 und den Lamellen 12 ebenfalls einen Sichtschutz. Durch diesen mechanischen Sichtschutz ist in der Regel ein Blick von außen auf die UV-Lampen 9 verhindert. Dennoch ist es möglich, dass durch Reflexionen Strahlung der UV-Lampen 9 aus dem Innenraum I durch den Einlass E oder den Auslass A gelangen könnte. Um dies zuverlässig zu verhindern ist an der Innenseite der Kammer K im Bereich des Einlasses E und/oder des Auslasses A eine absorbierende Oberfläche O an den Bauteilen in diesen Bereichen angeordnet. Die absorbierende Oberfläche O befindet sich somit im Einlass E auf den Lamellen 5 und 7 sowie der Leiste mit den Ventilatoren 6 und den entsprechend freien Stellen der Seitenwände 1 und 1', der Bodenwand 2 sowie der Deckwand 13. Im Bereich des Auslasses A ist die absorbierende Oberfläche O an den Lamellen 12, der Leitwand 8.1 sowie der letzten Leitwand 8 auf der dem Auslass A zugewandten Seite und den entsprechenden freien Stellen der Seitenwände 1 und 1', der Bodenwand 2 sowie der Deckwand 13 angeordnet. Diese absorbierende Oberfläche O verhindert eine Reflexion der Strahlung der UV-Lampen 9 aus der Kammer K. Die absorbierende Oberfläche O ist mit einer entsprechenden Schraffur angedeutet. Während somit der Innenraum I mit reflektierenden Oberflächen R ausgestattet ist, um die Wirkung der UV-Lampen 9 zu maximieren, wird in den Bereichen des Einlasses E und des Auslasses A durch die absorbierende Oberfläche O die weitere Reflexion der Strahlen verhindert. Personen, welche sich in der Nähe der Vorrichtung bzw. der Kammer K befinden, werden hierdurch geschützt, da keine Strahlung aus der Vorrichtung entweichen kann und die Personen schädigen könnte.

In Figur 2 ist eine Seitenansicht auf die Kammer K gemäß Figur 1 dargestellt, bei welcher die in Figur 1 unten dargestellte Seitenwand 1 entfernt wurde. Aus dieser Darstellung wird ersichtlich, dass sich die Leitwände 8 und 8.1 über die komplette Höhe zwischen Bodenwand 2 und Deckwand 13 erstrecken. Sie schließen dementsprechend den Strömungsweg ab, sodass die Luft entlang des kompletten Strömungswegs mäanderförmig durch den Innenraum I strömen muss und nicht über die Leitwand 8 hinweg abkürzen kann. Damit ist sichergestellt, dass die komplette Luft, welche durch die Vorrichtung strömt, gleichermaßen bestrahlt wurde.

Aus der Darstellung dieser Figur 2 ist weiterhin ersichtlich, dass die Lamellen 5, 7 und 12 geneigt sind. Hierdurch ist die Strömungsrichtung zum Einsaugen der Luft am Einlass E sowie zum Ausblasen der Luft am Auslass A beeinflussbar. Sind die Lamellen 5, 7 und/oder 12 in ihrer Neigung verstellbar ausgeführt, so ist einerseits die Richtung zum Einsaugen oder Ausblasen der Luft variierbar. Andererseits ist aber auch der Strömungswiderstand für die Luft veränderbar, sodass die durch die Vorrichtung hindurchströmende Luftmenge und Luftgeschwindigkeit einstellbar ist. Die Luftgeschwindigkeit und die Luftmenge sind darüber hinaus noch durch eine einstellbare Drehzahl der Ventilatoren 6 veränderbar. Durch beide Maßnahmen ist es möglich die Dauer festzulegen, für welche die Luft sich in dem Innenraum I der Kammer K befinden wird. Durch eine Verlängerung dieser Zeitdauer wird die Bestrahlung der Luft intensiver und damit auch die Desinfektion und Sterilisation der Luft verbessert.

Weiterhin ist auch in Figur 2 zu erkennen, dass im Bereich des Einlasses E und des Auslasses A die dort vorhandenen Bauteile der Kammer K mit einer absorbierenden Oberfläche O versehen sind. Diese absorbierende Oberfläche O kann entweder durch eine Farbe, beispielsweise eine matte, vorzugsweise strukturierte, schwarze Farbe, erzeugt werden. Es ist aber auch möglich, dass die absorbierende Oberfläche O mit anderen Beschichtungen oder Legierungen der Materialien der Bauteile in diesen Bereichen E und A erzeugt wird. Im Gegensatz zu diesen absorbierenden Oberflächen O in den Bereichen des Einlasses E und des Auslasses A ist die Oberfläche R der Bauteile im Innenraum I reflektierend ausgestaltet. Dies kann beispielsweise durch Aluminium, vorzugsweise poliertes Aluminium, für die Seitenwände 1 und die Leitwände 8 erzeugt werden.

Neben der oben detailliert beschriebenen Kammer K weist die Vorrichtung nicht dargestellte Stromversorgungselemente für die Ventilatoren 6 und UV-Lampen 9 sowie Schalter zum Ein- und Ausschalten der Vorrichtung und Befestigungselemente zum Befestigen der Vorrichtung, beispielsweise an einer Decke eines Zimmers, auf.

Die vorliegende Erfindung ist nicht auf die dargestellten und beschriebenen Ausführungsbeispiele beschränkt. Abwandlungen im Rahmen der Patentansprüche sind ebenso möglich wie eine Kombination der Merkmale, auch wenn diese in unterschiedlichen Ausführungsbeispielen dargestellt und beschrieben sind.

### Bezugszeichenliste

- 1: Seitenwand
- 1': Seitenwand
- 2: Bodenwand
- 3: Pfeil Eintrittsrichtung
- 4: Pfeil Austrittsrichtung
- 5: Lamellen
- 6: Ventilator
- 7: Lamellen
- 8: Leitwand
- 8.1: Leitwand
- 9: UV-Lampe
- 10: Abwinkelung
- 11: Verwirbelungskante
- 12: Lamellen
- 13: Deckwand

- A: Auslass
- E: Einlass
- H: Bestrahlungsdosis
- I: Innenraum
- K: Kammer
- O: absorbierende Oberfläche
- R: reflektierende Oberfläche
- S: Strömungsweg

## Patentansprüche

1. Verfahren zum Desinfizieren und Sterilisieren von Luft,
- wobei die Luft durch einen Einlass (E) in einen Innenraum (I) einer Kammer (K) einer Vorrichtung eingesaugt wird,
- die Luft in dem Innenraum (I) in einem mäandrierenden Strömungsweg (S) entlang von mehreren UV-Lampen (9) strömt und von den UV-Lampen (9) bestrahlt wird und
- anschließend den Innenraum (I) der Kammer (K) durch einen Auslass (A) wieder verlässt,
**dadurch gekennzeichnet, dass**
- die Strahlung der UV-Lampen (9) am Austreten aus der Kammer (K) durch eine mechanische Abschirmung und durch absorbierende Oberflächen (O) im Bereich des Einlasses (E) und/oder des Auslasses (A) gehindert wird.

2. Verfahren nach dem vorherigen Anspruch, **dadurch gekennzeichnet, dass** die Luft in dem Innenraum (I) mehr als 1 Sekunde, vorzugsweise mehr als 1,75 Sekunden verweilt.

3. Verfahren nach einem oder mehreren der vorherigen Ansprüche,
**dadurch gekennzeichnet, dass** die UV-Lampen (9) als UVC-Lampen (9) mit einer Wellenlänge zwischen 100 bis 280 nm, vorzugsweise zwischen 220 bis 280 nm, strahlen.

4. Verfahren nach einem oder mehreren der vorherigen Ansprüche,
**dadurch gekennzeichnet, dass** in der Vorrichtung eine Bestrahlungsdosis (H) von mindestens 50 J/m², vorzugsweise von mehr als 110 J/m² erzeugt wird.

5. Verfahren nach einem oder mehreren der vorherigen Ansprüche,
**dadurch gekennzeichnet, dass** die Luft in dem Innenraum (I) verwirbelt wird.

6. Vorrichtung zum Desinfizieren und Sterilisieren von Luft,
- die eine Kammer (K) mit Seitenwänden (1, 1'), einer Bodenwand (2) und einer Deckwand (13) aufweist, wobei die Kammer (K) einen Innenraum (I), einen Einlass (E) und einen Auslass (A) umfasst,
- der Kammer (K) ein Gebläse zugeordnet ist,
- in dem Innenraum (I) der Kammer (K) aus zumindest einer Leitwand (8) ein Labyrinth gebildet ist, das einen mäandrierenden Strömungsweg (S) für die Luft bildet,
- in dem Strömungsweg (S) mehrere UV-Lampen (9) angebracht sind,
- wobei die UV-Lampen (9) in Richtung des Strömungsweges (S) ausgerichtet sind, so dass die Luft entlang der UV-Lampen (9) strömt, **dadurch gekennzeichnet,**
- **dass** der Kammer (K) ein Sichtschutz zugeordnet ist, um eine Sicht auf die UV-Lampen (9) durch den Einlass (E) und den Auslass (A) zu verhindern und
- **dass** eine Innenseite der Kammer (K) im Bereich des Einlasses (E) und/oder des Auslasses (A) eine absorbierende Oberfläche (O) aufweist, um eine Reflexion der Strahlung der UV-Lampen (9) aus der Kammer (K) zu verhindern.

7. Vorrichtung nach dem vorherigen Anspruch, **dadurch gekennzeichnet, dass** die Innenseite der Kammer (K) im Bereich des Innenraums (I) eine reflektierende Oberfläche (R) aufweist.

8. Vorrichtung nach einem oder mehreren der vorherigen Ansprüche,
**dadurch gekennzeichnet, dass** die UV-Lampen (9) zwischen benachbarten Leitwänden (8) angeordnet sind.

9. Vorrichtung nach einem oder mehreren der vorherigen Ansprüche,
**dadurch gekennzeichnet, dass** sich die Leitwand (8) zwischen der Bodenwand und der Deckwand erstreckt.

10. Vorrichtung nach einem oder mehreren der vorherigen Ansprüche,
**dadurch gekennzeichnet, dass** die Leitwand (8) zwei Enden aufweist, wobei eines der Enden an eine der Seitenwände (1, 1') anschließt und das andere Ende frei in den Strömungsweg (S) ragt.

11. Vorrichtung nach einem oder mehreren der vorherigen Ansprüche,
**dadurch gekennzeichnet, dass** zumindest eines der Enden der Leitwand (8) eine Abwinkelung (10) aufweist un/oder das freie Ende der Leitwand (8) eine Verwirbelungskante (11) aufweist.

12. Vorrichtung nach einem oder mehreren der vorherigen Ansprüche,
**dadurch gekennzeichnet, dass** an einer der Seitenwände (1, 1') das Gebläse, vorzugsweise mehrere Ventilatoren (6) umfassend, angeordnet ist.

13. Vorrichtung nach einem oder mehreren der vorherigen Ansprüche,
**dadurch gekennzeichnet, dass** am Einlass (E) und/oder am Auslass (A) Lamellen (5, 6, 12) angeordnet sind, um die Luft gleichmäßig in und/oder aus dem Innenraum (I) zu fördern und die Lamellen (5, 6, 12) vorzugsweise eine absorbierende Oberfläche (O) aufweisen.

14. Vorrichtung nach einem oder mehreren der vorherigen Ansprüche,
**dadurch gekennzeichnet, dass** die UV-Lampen (9) UVC-Lampen (9) mit einer Wellenlänge zwischen 100 bis 280 nm, vorzugsweise zwischen 220 bis 280 nm sind.

15. Vorrichtung nach einem oder mehreren der vorherigen Ansprüche,
**dadurch gekennzeichnet, dass** die UV-Lampen (9), insbesondere die UVC-Lampen (9), in der Vorrichtung eine Bestrahlungsdosis (H) von mindestens 50 J/m², vorzugsweise von mehr als 110 J/m² erzeugen.
